# FASCICULE DE BREVET EUROPEEN

(11) **EP 1 102 747 B1**
(45) Date de publication et mention de la délivrance du brevet: **18.06.2003**
(21) Numéro de dépôt: 99936672.7
(22) Date de dépôt: 03.08.1999
(51) Int. Cl.: C07D 211/58, A61K 31/445

(54) **FORMES CRISTALLINES DU OSANETANT**
KRISTALLFORMEN VON OSANETANT
CRYSTALLINE FORMS OF OSANETANT

(30) Priorité: 05.08.1998 FR 9810107
(43) Date de publication de la demande: 30.05.2001
(73) Titulaire: SANOFI-SYNTHELABO, 75013 Paris (FR)
(72) Inventeur: ALCADE, Alain, 31100 Toulouse (FR); ANNE-ARCHARD, Gilles, 31000 Toulouse (FR); GROSCLAUDE, Patrick, 31450 Belberaud (FR); MONNIER, Olivier, 34560 Villeveyrac (FR); ROCHE, Jérôme, 34730 Prades Le Lez (FR)
(74) Mandataire: Thouret-Lemaitre, Elisabeth
(86) Numéro de dépôt international: FR9901914
(87) Numéro de publication internationale: WO00007987

(56) Documents cités:
- EP-A- 0 673 928
- WO-A-98/05640
- CHEN, HUAI. G. ET AL: "A practical and scalable synthesis of SR 142801, a tachykinin NK3 antagonist" BIOORG. MED. CHEM. LETT. (1997), 7(5), 555-560 , XP002055505
- GIARDINA, GIUSEPPE A. M. ET AL: "A reliable and efficient synthesis of SR 142801" BIOORG. MED. CHEM. LETT. (1996), 6(19), 2307-2310 , XP000197497

## Description

La présente invention concerne deux formes cristallines différentes du (R)-(+)-N-[[3-[1-benzoyl-3-(3,4-dichlorophényl)pipéridin-3-yl]prop-1-yl]-4-phénylpipéridin-4-yl]-N-méthylacétamide et un procédé pour leur préparation. Le (R)-(+)-N-[[3-[1-benzoyl-3-(3,4-dichlorophényl)pipéridin-3-yl]prop-1-yl]-4-phénylpipéridin-4-yl]-N-méthylacétamide, ci-après désigné par sa Dénomination Commune Internationale "osanétant", est le premier antagoniste du récepteur NK-3 décrit en littérature, dont la préparation, notamment sous forme de chlorhydrate, est illustrée dans EP-A-673 928.

Selon ce document, l'osanétant est préparé en faisant réagir la N-méthyl-N-(4-phénylpipéridin-4-yl)acétamide avec la 1-benzoyl-3-(3,4-dichlorophényl)-3-(méthanesulfonyloxyprop-1-yl)-pipéridine et en transformant l'osanétant ainsi obtenu en son chlorhydrate. On a constaté que le chlorhydrate d'osanétant est isolé sous forme d'un solide amorphe difficilement purifiable. Ce produit contient des impuretés provenant des étapes de synthèse précédentes.

Pour obtenir l'osenétant sous forme pure, on peut utiliser la chromatographie préparative à partir de l'osanétant base.

Par ailleurs, il a été trouvé qu'en isolant l'osanétant sous forme de benzènesulfonate, par exemple sous forme de solvate de son benzènesulfonate avec la 4-méthyl-2-pentanone, on obtient un produit extrêmement pur qui donne facilement l'osanétant chimiquement pur.

Il a été aussi trouvé qu'en cristallisant l'osanétant de façon convenable, on obtient deux formes cristallines différentes. De préférence, l'osanétant de départ est chimiquement pur. Par chimiquement pur, on entend que l'osanétant contient moins de 20 % d'impuretés et de préférence moins de 10 %.

Plus particulièrement, il a été trouvé un procédé de cristallisation de l'osanétant caractérisé en ce que :
i) l'osanétant est cristallisé d'un mélange éthanol/eau ou de l'isopropanol pour donner la Forme cristalline I ;
ii) l'osanétant est cristallisé d'un mélange éthanol/éther isopropylique/eau pour donner la Forme cristalline II.

Préférentiellement, selon la présente invention, l'osanétant à cristalliser est préparé par neutralisation de son benzènesulfonate.

Ainsi, selon un de ses aspects, la présente invention concerne un procédé pour la préparation de l'osanétant caractérisé en ce que l'on prépare le benzènesulfonate d'osanétant puis on le neutralise avec une base.

Le benzènesulfonate d'osanétant peut être obtenu à partir d'un sel d'osanétant. Ainsi, selon le procédé de la présente invention :
a) on neutralise un sel d'osanétant avec une base et ensuite on traite la solution d'osanétant ainsi obtenue avec de l'acide benzènesulfonique ;
b) on neutralise le benzènesulfonate d'osanétant ainsi obtenu avec une base.

Ce procédé s'applique en particulier à la préparation de l'osanétant pur à partir du chlorhydrate d'osanétant. Ainsi, selon un aspect particulier du procédé de la présente invention :
a) on neutralise le chlorhydrate d'osanétant avec une base et, ensuite, on traite la solution d'osanétant ainsi obtenue avec de l'acide benzènesulfonique ; et
(b) on neutralise avec une base le benzènesulfonate ;
puis on isole l'osanétant pur ainsi obtenu.

A l'étape (a), le chlorhydrate d'osanétant est traité avec une base telle que la soude, la potasse ou l'ammoniaque dans l'eau en présence d'un solvant, de préférence la 4-méthyl-2-pentanone, puis l'osanétant est avantageusement isolé sous forme de benzènesulfonate en traitant la solution d'osanétant ainsi obtenue avec de l'acide benzènesulfonique, ce qui permet l'obtention d'un produit extrêmement pur. La salification étant généralement conduite dans la 4-méthyl-2-pentanone et ce solvant tendant à donner des solvates, le benzènesulfonate d'osanétant peut être sous forme de solvate avec la 4-méthyl-2-pentanone, de préférence avec 0,25 mole de celle-ci. Les rendements sont pratiquement quantitatifs, généralement supérieurs à 94 %.

Dans l'étape (b) le benzènesulfonate d'osanétant, ou dans le cas où dans l'étape a) on utilise comme solvant la 4-méthyl-2-pentanone, son solvate avec la 4-méthyl-2-pentanone, est neutralisé avec une base telle que l'hydroxyde de sodium ou de potassium ou l'ammoniaque et l'osanétant est isolé en général à partir de ses solutions dans un alcool, de préférence l'éthanol ou l'isopropanol, éventuellement en mélange avec d'autres solvants, d'où l'osanétant cristallise.

La neutralisation de l'étape b) est en général effectuée dans un solvant halogéné tel que le dichlorométhane, le 1,2-dichloroéthane ou le 1,1,1-trichloroéthane, en mélange avec de l'eau. La neutralisation peut également être réalisée dans un mélange éthanol-eau afin d'obtenir directement l'osanétant sous Forme cristalline I.

Selon un autre de ses aspects, la présente invention concerne un procédé pour la cristallisation de l'osanétant caractérisé en ce que :
- soit on ajoute de l'eau à une solution d'osanétant dans l'éthanol et on chauffe à une température inférieure ou égale à la température de reflux du solvant, de préférence 60 à 75°C, puis on refroidit pour obtenir l'osanétant - Forme cristalline I ;
- soit on chauffe une solution d'osanétant dans l'isopropanol à une température inférieure ou égale à la température de reflux du solvant, de préférence 60 à 75°C, et on refroidit pour obtenir l'osanétant - Forme cristalline I ;
- soit on ajoute de l'éther isopropylique et de l'eau à une solution d'osanétant dans l'éthanol, on chauffe, de préférence à reflux du solvant, et on refroidit pour obtenir l'osanétant - Forme cristalline II.

Dans le procédé de cristallisation de l'invention on peut utiliser l'osanétant pur obtenu par n'importe quel procédé. Avantageusement, on utilise l'osanétant obtenu par le procédé de préparation décrit ci-dessus.

Ainsi, par exemple, lorsque la neutralisation du benzènesulfonate d'osanétant selon l'étape (b) du procédé ci-dessus est terminée, l'osanétant peut être mis en solution dans l'éthanol ou l'isopropanol et peut être isolé soit sous sa Forme cristalline I, soit sous sa Forme cristalline II.

Selon une variante de mise en oeuvre du procédé de cristallisation de l'invention, on peut, pour isoler la Forme cristalline I, ajouter de l'eau à la solution éthanolique contenant l'osanétant, par exemple dans une proportion de 40 % d'eau et 60 % d'éthanol ; puis chauffer à 60-75°C et laisser refroidir sous forte agitation jusqu'à 20-25°C. L'osanétant - Forme cristalline I cristallise et l'on peut avantageusement accélérer la cristallisation en ensemençant avec des cristaux d'osanétant-Forme cristalline 1 ; on augmente alors la température jusqu'à 45-50°C, refroidit lentement jusqu'à 0°C et on maintient à cette température jusqu'à ce que la cristallisation soit complète. L'osanétant - Forme cristalline I est alors isolé par filtration, lavé et séché.

Selon une alternative, on peut pour isoler la Forme cristalline I, ajouter de l'eau à la solution éthanolique contenant l'osanétant, chauffée à 60-75°C, et laisser refroidir lentement sous forte agitation à une température voisine de 40°C ; ensemencer le milieu avec des cristaux d'osanétant - Forme cristalline I, continuer à refroidir lentement jusqu'à 20-25°C, et maintenir à cette température pendant quelques heures ; réchauffer ensuite lentement la suspension d'osanétant formée jusqu'à 45-50°C, maintenir à cette température quelques heures, puis refroidir lentement jusqu'à 20°C et enfin filtrer les cristaux formés.

Selon une autre variante de mise en oeuvre du procédé de cristallisation de l'invention on peut également, pour isoler la Forme cristalline I, chauffer une solution isopropanolique d'osanétant à 60-80°C, de préférence 60-75°C, puis la refroidir sous agitation, préférentiellement avec une rampe de refroidissement jusqu'à 0°C. L'osanétant - Forme I est alors isolé par filtration, lavé et séché.

Avantageusement, on refroidit la solution isopropanolique d'osanétant jusqu'à une température comprise entre 0°C et 50°C, préférentiellement 35-50°C puis, on amorce la cristallisation en ensemençant et le cas échéant on continue à refroidir jusqu'à 0°C puis on maintient cette température jusqu'à ce que la cristallisation soit complète.

La concentration de l'onasétant dans l'isopropanol est préférentiellement comprise entre 200 g et 350g/litre, plus particulièrement entre 250 g et 300 g/litre.

Lorsque la cristallisation est effectuée dans un volume réactionnel de 0,5 litre à 2 litres, l'agitation est effectuée préférentiellement avec des vitesses comprises entre 200 et 600 tours/minute. On peut par exemple utiliser comme agitateur un mobile d'agitation à palette, type "impeller".

Afin d'effectuer un refroidissement contrôlé, on utilise préférentiellement une rampe de refroidissement linéaire de -10 à -30°C / heure, par exemple de -20°C / heure.

Pour isoler la Forme cristalline II, on peut avantageusement ajouter de l'éther isopropylique à la solution éthanolique contenant l'osanétant, puis chauffer à reflux, ensuite ajouter de l'éther isopropylique et 0,5 à 3% d'eau. Par refroidissement jusqu'à 40-50°C, la Forme cristalline II de l'osanétant cristallise ; préférentiellement on refroidit la suspension jusqu'à 25 °C et on isole la Forme cristalline II par filtration, lavage et séchage.

Lorsque les deux formes cristallines de l'osanétant sont obtenues à partir de solutions éthanoliques du produit, il est possible de passer facilement d'une forme cristalline à l'autre par chauffage à reflux dans des mélanges éthanol/eau ou éthanol/éther isopropylique/eau dans les conditions indiquées ci-dessus.

Plus particulièrement, il est possible de passer de l'osanétant-Forme cristalline I à l'osanétant-Forme cristalline II en chauffant à reflux un mélange de Forme cristalline I dans un mélange d'éthanol/éther isopropylique, environ 1/1 (v/v), en ajoutant de l'eau et de l'éther isopropylique et en refroidissant comme décrit ci-dessus. De même, il est possible de passer de l'osanétant-Forme cristalline II à l'osanétant-Forme cristalline I en chauffant à reflux la Forme cristalline II dans un mélange éthanol/eau, environ 1/1 (v/v) et en refroidissant comme décrit ci-dessus.

Dans tous les cas, il est préférable de filtrer les solutions chaudes, avant le refroidissement, pour éliminer des germes de cristallisation parasites éventuellement présents.

Ainsi, selon un mode opératoire avantageux, le procédé pour la préparation de l'osanétant selon la présente invention est caractérisé en ce qu'on conduit l'étape (a) comme illustré ci-dessus, puis, (b) on neutralise le benzènesulfonate d'osanétant ainsi obtenu, ou un solvate de celui-ci avec la 4-méthyl-2-pentanone, avec un hydroxyde alcalin dans un solvant halogéné choisi parmi le dichlorométhane, le 1,2-dichloroéthane ou le 1,1,1,-trichloroéthane, et
- soit on ajoute de l'éthanol, on distille le solvant halogéné par distillation azéotropique et on ajoute de l'eau à la solution éthanolique contenant l'osanétant à une température de 60 à 75°C et on laisse cristalliser l'osanétant-Forme cristalline I ;
- soit on ajoute de l'isopropanol, on distille le solvant halogéné par distillation azéotropique puis, on chauffe à une température de 60-80°C et on laisse cristalliser l'osanétant - Forme cristalline I ;
- soit on ajoute de l'éthanol, on distille le solvant halogéné par distillation azéotropique et on ajoute de l'éther isopropylique et de l'eau à la solution éthanolique de l'osanétant, puis on chauffe à reflux et on laisse cristalliser l'osanétant -Forme cristalline II.

Selon un aspect particulier de la présente invention, l'osanétant-Forme cristalline I est préparé par le procédé qui comprend les étapes consistant à chauffer à 60-75°C une solution éthanolique d'osanétant, à ajouter de l'eau, à refroidir jusqu'à 20-25°C puis, soit à amorcer la cristallisation, soit à attendre que les premiers cristaux apparaissent et, ensuite, à augmenter la température jusqu'à 45-50°C, à refroidir jusqu'à à 0 °C et à maintenir cette température jusqu'à ce que la cristallisation soit complète.

Selon un aspect préféré de la présente invention, l'osanétant - Forme I peut être obtenu en utilisant le procédé qui comprend les étapes consistant à :
- chauffer entre 60 et 75°C une solution d'osanétant dans de l'isopropanol à la concentration de 200-350 g/l, de préférence 250-300 g/l ;
- refroidir la solution jusqu'à une température comprise entre 0° et 50°C, par exemple 35-50°C, de préférence 40°C avec une rampe de refroidissement linéaire de -10°C à -30°C, de préférence -20°C/heure, sous agitation ;
- ensemencer le milieu avec 2 à 10 %, de préférence 5 % d'osanétant - Forme I ;
- refroidir le milieu jusqu'à 0°C avec une rampe de refroidissement linéaire de -10°C à -30 °C / heure, de préférence -20°C/heure, et à maintenir à cette température jusqu'à la cristallisation complète ;
- isoler les cristaux formés.

L'application des conditions opératoires selon le procédé préféré de la présente invention permet d'obtenir un rendement de cristallisation en osanétant - Forme I, supérieur à 90% dans un temps inférieur à 10 heures.

Selon un autre aspect particulier de la présente invention, l'osanétant-Forme cristalline II est préparé par un procédé qui comprend les étapes qui consistent à chauffer à reflux une solution d'osanétant dans un mélange éthanol/éther isopropylique environ 1/1 (v/v), à ajouter de l'éther isopropylique environ et de l'eau (proportions par rapport au volume final : éther isopropylique environ 3,33 et eau 0,02 à 0,05), à laisser d'abord refroidir jusqu'à 40-50°C, par exemple environ 45°C, puis à amorcer la cristallisation, soit à attendre que les premiers cristaux apparaissent et à refroidir ensuite jusqu'à 20-25°C jusqu'à ce que la cristallisation soit complète.

Selon un autre de ses aspects, la présente invention a pour objet l'osanétant-Forme cristalline I et l'osanétant-Forme cristalline II, susceptibles d'être obtenus par le procédé illustré ci-dessus, notamment par les étapes (a) et (b), ainsi que par le procédé de cristallisation à partir d'une solution d'osanétant.

Plus particulièrement, selon cet aspect, la présente invention concerne :
- l'osanétant-Forme cristalline I, susceptible d'être obtenu :
   1) par le procédé comprenant les étapes qui consistent à chauffer à 60-75°C une solution éthanolique d'osanétant, à ajouter de l'eau, à refroidir jusqu'à 20-25°C puis, soit à amorcer la cristallisation, soit à attendre que les premiers cristaux apparaissent, et, ensuite à augmenter la température jusqu'à 45-50°C, à refroidir jusqu'à 0°C et on maintient à cette température jusqu'à ce que la cristallisation soit complète ;
   2) ou par le procédé comprenant les étapes qui consiste à chauffer à 60-80°C une solution d'osanétant dans l'isopropanol, à refroidir jusqu'à une température comprise entre 0°C et 50°C, préférentiellement 35-50°C puis, à amorcer la cristallisation, à refroidir jusqu'à 0°C et à maintenir à 0°C jusqu'à ce que la cristallisation soit complète ;
- l'osanétant-Forme cristalline II, susceptible d'être obtenu par le procédé comprenant les étapes qui consistent à chauffer à reflux un mélange d'osanétant et d'éthanol/éther isopropylique environ 1/1 (v/v), à ajouter de l'éther isopropylique et de l'eau dans des proportions par rapport au volume final de : éther isopropylique environ 3,33 et eau 0,02 à 0,05 (v/v), à laisser refroidir d'abord jusqu'à 40-50°C, puis soit à amorcer la cristallisation, soit à attendre que les cristaux apparaissent, à refroidir ensuite jusqu'à la température ambiante (20-25°C) et à maintenir à cette température jusqu'à ce que la cristallisation soit complète.

Les caractéristiques essentielles des nouvelles formes cristallines de l'osanétant ont été déterminées par analyse calorimétrique différentielle (DSC) donnant, par des thermogrammes obtenus avec un calorimètre PERKIN-ELMER, la température de fusion et l'enthalpie liée à ladite fusion.

La DSC a été réalisée en utilisant un appareil DSC 7 PERKIN-ELMER dont l'étalonnage a été effectué par rapport aux endothermes de fusion de l'indium et du plomb ou du cyclohexane. Pour cette analyse on a utilisé de 3 à 6 mg de produit en coupelle d'aluminium sertie et percée sur le couvercle, dans une zone de température de 50°C à 180°C, à une vitesse de chauffage de 10°C/minute, en utilisant l'azote à titre de gaz de balayage.

D'une façon générale dans la présente description, les constantes physiques ont été déterminées à l'aide d'échantillons des formes I et II dont la pureté est supérieure ou égale à 99,9 %.

La température de fusion et l'enthalpie de fusion constituent des caractéristiques essentielles pour identifier chaque forme cristalline.

Lesdites formes peuvent être aussi caractérisées par diffractométrie des rayons X de la poudre. Le profil de diffraction des rayons X de la poudre (angles de diffraction) a été établi avec un diffractomètre SIEMENS D 500 TT avec un générateur 40 kV, monochromateur arrière, source Cu Kα1 (λ = 1,5406 Å), portoir en silicium et dans un domaine de balayage de 4° à 40° à 1° par minute en 2 thêta de Bragg.

La Forme cristalline I de l'osanétant, qui présente :
- une température de fusion avec un pic dont le maximum est à 143,6°C ± 0,5°C
- une enthalpie de fusion de 68,5 ± 0,5 J/g, constitue un aspect préféré de la présente invention.

L'osanétant-Forme cristalline I a été aussi analysé par diffraction des rayons X de la poudre. L'étude qualitative des diffractogrammes a permis d'établir que cette forme cristalline présente des raies caractéristiques en 2 thêta de Bragg à environ 17,81° ; 11,04° et 16,84°.

La Forme cristalline II de l'osanétant qui présente :
- une température de fusion de 141,8 ± 0,5°C
- une enthalpie de fusion de 65,0 ± 0,5 J/g,
constitue un autre aspect préféré de la présente invention.

L'osanétant-Forme cristalline II a été aussi analysé par diffraction des rayons X de la poudre. L'étude qualitative des diffractogrammes a permi d'établir que cette forme cristalline présente des raies caractéristiques en 2 thêta de Bragg à à environ 18,35°; 18,58° et 18,97°.

Le fait de maîtriser la reproductibilité du procédé de fabrication de chacune des formes cristallines de l'osanétant permet de disposer de formes cristallines bien définies et est donc très avantageux pour l'utilisation de l'osanétant comme médicament et pour l'obtention des autorisations nécessaires pour la commercialisation dudit médicament.

Plus particulièrement, l'obtention d'un produit ayant une forme cristalline bien définie permet de préparer des formulations pharmaceutiques ayant une composition constante et reproductible, ce qui est particulièrement avantageux lorsque lesdites formulations sont destinées à l'administration orale.

Ainsi, selon un autre de ses aspects, la présente invention a pour objet une composition pharmaceutique contenant, en tant que principe actif, l'osanétant-Forme cristalline I ou l'osanétant-Forme cristalline II.

L'administration des formes cristallines de l'invention peut être convenablement effectuée par voie orale, parentérale, sublinguale, transdermique ou par inhalation. La quantité de principe actif à administrer dépend de la nature et de la gravité des maladies à traiter ainsi que du poids des malades. Néanmoins, le principe actif, administré en unité de dosage, est présent dans ladite unité de dosage en quantité de 0,5 à 500 mg, avantageusement de 1 à 250 mg, de préférence de 2 à 100 mg. Cette unité de dosage peut être administrée une à quatre fois par jour, de préférence une ou deux fois par jour.

Dans les formes unitaires des compositions pharmaceutiques de la présente invention, le principe actif est de préférence en mélange avec des excipients pharmaceutiques et il est administré aux animaux et aux êtres humains pour le traitement des maladies qui nécessitent un traitement basé sur l'administration d'un antagoniste des récepteurs NK-3, telles que, par exemple, celles indiquées dans EP-A-673928.

Les formes unitaires d'administration appropriées comprennent de préférence les formes par voie orale comme les comprimés, éventuellement sécables, les gélules, les poudres et les granules (pour lesquels l'unité de dosage peut être représentée par exemple par un sachet) ainsi que les formes d'administration sublinguale et buccale, les formes d'administration transdermique pouvant également être préparées en utilisant les nouvelles formes cristallines de l'invention en tant que principes actifs.

Lorsqu'on prépare une composition solide sous forme de comprimés, on mélange le principe actif avec un excipient pharmaceutique, tel que la gélatine, l'amidon, le lactose, le stéarate de magnésium, le talc, la gomme arabique. On peut enrober les comprimés de saccharose ou d'autres matières appropriées ou encore on peut les traiter de telle sorte qu'ils aient une activité prolongée ou retardée et qu'ils libèrent de façon continue une quantité prédéterminée de principe actif.

On obtient une préparation en gélules en mélangeant le principe actif avec un diluant et en versant le mélange ainsi préparé dans des gélules molles ou dures.

Le principe actif peut être également formulé sous forme de microcapsules, éventuellement avec un ou plusieurs supports ou additifs.

Dans les formes aérosol, le principe actif est administré par des dispositifs qui permettent l'absorption par voie respiratoire d'une unité de dosage.

Dans les compositions pharmaceutiques selon la présente invention, le principe actif peut être aussi sous forme de complexe d'inclusion dans des cyclodextrines, leurs éthers et leurs esters.

Enfin, le principe actif peut être associé à d'autres principes actifs, par exemple des bronchodilatateurs, des antitussifs ou des antihistaminiques.

Les EXEMPLES suivants illustrent l'invention.

### EXEMPLE 1

### (a) Osanétant benzènesulfonate solvate avec 0,25 mole de 4-méthyl-2-pentanone.

On prépare une suspension de 1,424 kg de chlorhydrate d'osanétant obtenu selon la demande de brevet EP-A-673 928 dans 2,5 litres d'eau et 8,64 litres de 4-méthyl-2-pentanone, on ajoute 0,32 kg de solution de NaOH à 30 %. Le mélange ainsi obtenu est chauffé à 80-85° C sous agitation pendant 15 minutes, puis la phase aqueuse est éliminée, la phase organique est lavée jusqu'à ce que le pH devienne inférieur à 8. On sèche par azéotropie et on refroidit la solution à 25°C, puis on ajoute, sous agitation une solution de 0,36 kg d'acide benzènesulfonique dans 1,15 l de 4-méthyl-2-pentanone. Après 15 heures d'agitation, on filtre le benzènesulfonate d'osanétant, solvaté avec 0,25 mole de 4-méthyl-2-pentanone ; F = 176 - 177°C (DSC) ; Rendement : 94,8 %

### (b) Osanétant

A un mélange de 1,64 kg du produit obtenu dans l'étape (a), de 4,92 l de dichlorométhane et de 3,28 l d'eau, on ajoute 0,3 kg d'une solution d'hydroxyde de sodium à 30 % tout en maintenant la température à environ 20°C, puis on décante et on extrait la phase aqueuse avec du dichlorométhane. Les phases organiques réunies sont lavées à l'eau jusqu'à pH inférieur à 7,5, puis elles sont séchées et concentrées par distillation de l'azéotrope dichlorométhane/eau.

À la solution concentrée ainsi obtenue, on ajoute 1,4 l d'éthanol, puis on élimine l'azéotrope dichlorométhane/éthanol tout en maintenant constant le volume de la solution par addition d'éthanol. On obtient 1260 g d'osanétant en solution dans l'éthanol.

### EXEMPLE 2

### Osanétant - Forme cristalline I.

On opère comme décrit dans l'EXEMPLE 1, la solution éthanolique obtenue, constituée par 3,3 kg de solution contenant l'osanétant dans l'éthanol, est diluée avec 1,76 kg d'éthanol, puis chauffée à 70°C. A cette température, on ajoute progressivement 3,2 l d'eau et le mélange est refroidi lentement à 20-25°C sous forte agitation. Dès que les premiers cristaux apparaissent, on agite pendant 15 heures, puis on chauffe à 45-50°C et on laisse le mélange pendant 3 heures à cette température. Le mélange est refroidi à 0° C et laissé à cette température pendant 15 heures. On filtre le précipité, on le lave par un mélange éthanol/eau 60/40 (v/v) préalablement refroidi à 0°C, et on le sèche sous vide à 80°C.

Dans ces conditions, on a aussi obtenu l'osanétant - Forme cristalline I, de pureté HPLC de 99,9 %, avec un rendement de 90 % dans l'étape (b).

A l'analyse calorimétrique différentielle (DSC), l'osanétant Forme cristalline I obtenu dans cette préparation a présenté
- une température de fusion de 143,6 °C
- une enthalpie de fusion de 68,5 J/g

A l'analyse de diffraction des rayons X de la poudre, avec un diffractomètre SIEMENS D 500 TT dans les conditions données ci-dessus, l'osanétant - Forme cristalline I obtenu dans cette préparation présente des raies caractéristiques des angles 2 thêta de Bragg de 17,81°, 11,04° et 16,84°.

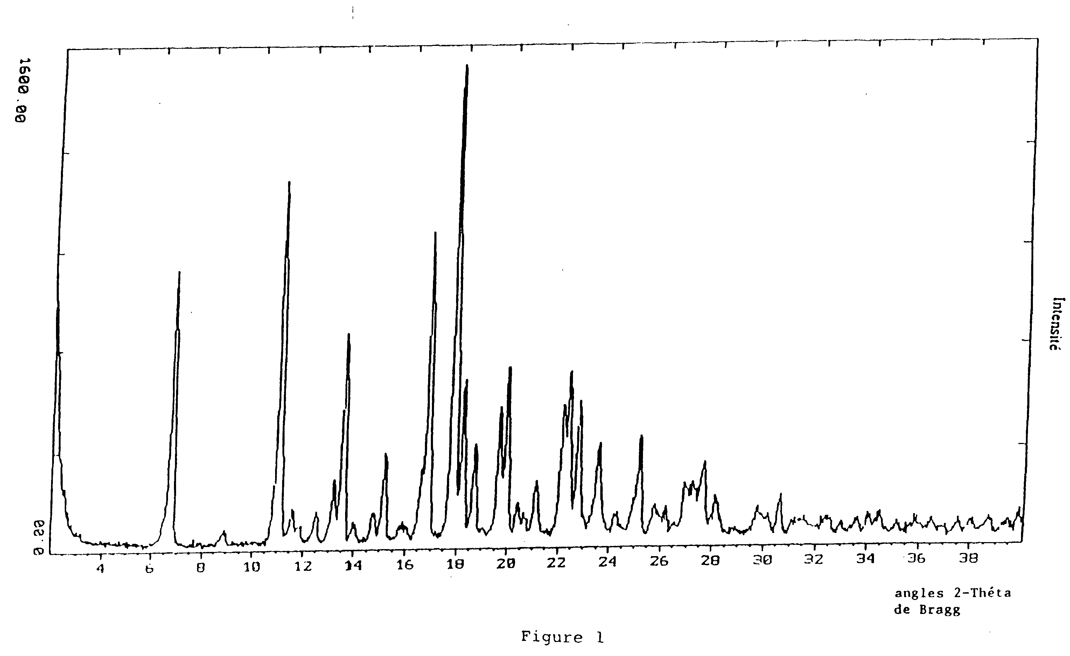

Le diffractogramme relatif est consigné dans la figure 1.

Le profil de diffraction des rayons X de la poudre (angles de diffraction) de l'osanétant Forme cristalline I de cette préparation est donné par les raies significatives rassemblées dans le TABLEAU 1, avec l'intensité relative, exprimée en pourcentage par rapport à la raie la plus intense.

**TABLEAU 1**

| OSANETANT - FORME CRISTALLINE I | |
|---|---|
| *Bandes de diffraction* *(angles 2* θ *de Bragg)* | *Intensité relative* |
| 17,81 | 100 |
| 11,04 | 77,0 |
| 16,84 | 65,8 |
| 6,75 | 58,3 |
| 13,53 | 44,5 |
| 19,92 | 37,4 |
| 22,31 | 36,4 |
| 18,19 | 34,9 |
| 22,73 | 30,6 |
| 19,60 | 29,45 |
| 22,15 | 28,2 |
| 25,10 | 23,3 |
| 23,49 | 22,1 |
| 18,66 | 22,1 |
| 15,14 | 20,4 |

### EXEMPLE 3

### Osanétant - Forme cristalline II.

On chauffe à reflux sous atmosphère d'azote et sous agitation le mélange de 100 g d'osanétant - Forme cristalline I, de 92 ml d'éthanol et de 92 ml d'éther isopropylique, puis on ajoute 2,96 g d'eau et 306 ml d'éther isopropylique.

On filtre la solution pour éliminer toutes traces de germes parasites, puis on la refroidit à 43 - 47° C sous agitation et on la maintient pendant 5 - 6 heures à cette température. Dans ces conditions, l'osanétant - Forme cristalline II cristallise. On refroidit alors à environ 25 ° C et on maintient le mélange sous agitation pendant 3 heures. On filtre les cristaux ainsi séparés, on les lave avec 100 ml d'un mélange éthanol / éther isopropylique 19/81 (v/v) et on les sèche sous vide à 65°C.

Dans une préparation dans ces conditions on a obtenu 73 g d'osanétant - Forme cristalline II de pureté HPLC de 99,9 %.

A l'analyse calorimétrique différentielle (DSC), l'osanétant - Forme cristalline II obtenu dans cette préparation a présenté :
- une température de fusion de 141,8°C
- une enthalpie de fusion de 65,0 J/g.

A l'analyse de diffraction des rayons X de la poudre, avec un diffractomètre SIEMENS D 500 TT dans les conditions données ci-dessus, l'osanétant - Forme cristalline II obtenu dans cette préparation présente des raies caractéristiques des angles 2 thêta de Bragg de 18,35°, 18,58° et 18,97°.

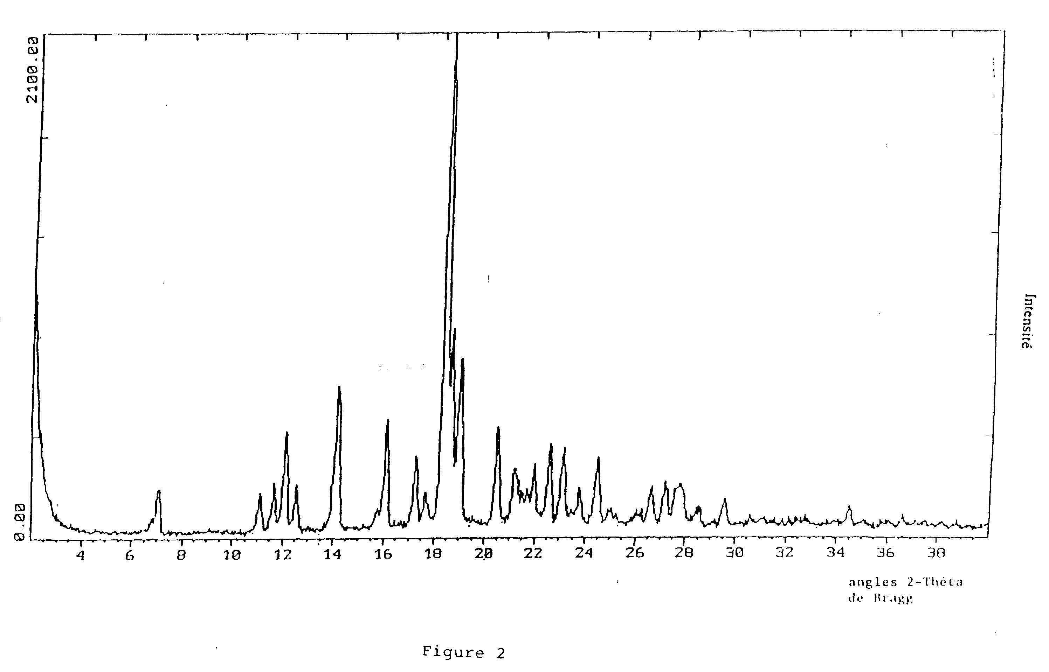

Le diffractogramme relatif est consigné dans la figure 2.

Le profil de diffraction des rayons X de la poudre (angles de diffraction) de l'osanétant - Forme cristalline II de cette préparation est donné par les raies significatives rassemblées dans le TABLEAU 2, avec l'intensité relative exprimée en pourcentage par rapport à la raie la plus intense.

**TABLEAU 2**

| OSANETANT - FORME CRISTALLINE II | |
|---|---|
| *Bandes de diffraction* *(angles 2* θ *de Bragg)* | *Intensité relative* |
| 18,35 | 100 |
| 18,58 | 39,30 |
| 18,97 | 35,49 |
| 14,09 | 30,2 |
| 16,05 | 23,49 |
| 20,47 | 21,87 |
| 12,05 | 21,2 |
| 22,54 | 18,48 |
| 23,06 | 17,53 |
| 17,21 | 16,01 |
| 24,44 | 15,63 |
| 21,94 | 14,34 |
| 21,17 | 13,53 |
| 11,6 | 10,86 |
| 27,17 | 10,81 |

### EXEMPLE 4

### Osanétant - Forme cristalline II.

On opère comme décrit dans l'EXEMPLE 1, mais la solution éthanolique obtenue à la fin de l'étape (b), constituée par 3,3 kg de solution contenant l'osanétant dans l'éthanol, est concentrée à 2,2 kg puis diluée avec 1,175 l d'éther isopropylique, puis on chauffe à reflux sous agitation et on ajoute au mélange 37,9 ml d'eau et 3,9 l d'éther isopropylique. On filtre la solution ainsi obtenue, on la refroidit à 45° C sous agitation, on amorce la cristallisation et on maintient à cette température pendant 5 heures et 30 minutes. Le mélange est refroidi à 25° C et maintenu sous agitation pendant 3 heures à cette température. On filtre le produit ainsi cristallisé, on le lave avec un mélange éthanol/éther isopropylique 19/81 (v/v), et on le sèche sous vide à 65° C jusqu'à poids constant. On obtient ainsi l'osanétant - Forme cristalline II.

### EXEMPLE 5

### Osanétant - Forme cristalline I

A une solution de 100 g d'osanétant - Forme cristalline II (obtenu comme décrit dans l'EXEMPLE 3) dans 378 ml d'éthanol, chauffée à 70° C on ajoute progressivement 252 ml d'eau, puis on refroidit lentement à 20 - 25° C sous forte agitation, on amorce la cristallisation et on maintient le mélange sous agitation pendant 15 heures. On chauffe progressivement à 45-50° C et on laisse le mélange à cette température pendant 3 heures. On refroidit lentement à 0° C, on maintient cette température pendant 15 heures, puis on filtre le produit ainsi précipité, on le lave avec un mélange éthanol / eau 60/40 (v/v) préalablement refroidi à 0° C et on sèche sous vide à 80° C. On obtient ainsi l'osanétant - Forme cristalline I.

### EXEMPLE 6

### Osanétant - Forme cristalline I

On opère comme décrit à l'EXEMPLE 1, étape a), et la première partie de l'étape b).

Après distillation de l'azéotrope dichlorométhane/eau, on ajoute de l'isopropanol puis on élimine l'azéotrope dichlorométhane/isopropanol en maintenant constant le volume par addition d'isopropanol. La solution d'isopropanol ainsi obtenue est concentrée par distillation sous pression réduite jusqu'à contenir 225,4 g d'osanétant dans 680 ml, soit une concentration de 260 g d'osanétant/litre. Cette solution, dont la température est voisine de 60°C, est agitée à 400 tours/minutes avec un mobile d'agitation à palette de type "impeller" de 7 cm de diamètre de rotation pour un volume réactionnel de 0,5 litre dans un réacteur de 2 litres. Simultanément cette solution est refroidie avec une rampe de refroidissement linéaire de -20°C/heure. A la température de 40°C, on ensemence le milieu avec 5 % en poids de cristaux d'osanétant - Forme I et on continue à refroidir la solution obtenue jusqu'à 0°C, avec la rampe de refroidissement de -20°C/heure. On maintient le milieu à 0°C pendant 6 heures puis on filtre les cristaux formés. On les lave avec de l'isopropanol puis on sèche sous vide à 80°C. On obtient ainsi l'osanétant - Forme cristalline I avec un rendement de 93 %.

### EXEMPLE 7 :

### Osanétant - Forme cristalline I

A une suspension de 20 g d'osanétant benzènesulfonate, solvate avec 0,25 mole de 4-méthyl-2-pentanone, obtenu à l'EXEMPLE 1, étape a), dans 60 ml d'éthanol à 100 %, on ajoute 1,2 g de NaOH dans 20 ml d'éthanol à 100 %. Après une heure sous agitation à température ambiante, on filtre le benzènesulfonate de sodium formé et on le lave par 10 ml d'éthanol à 100 %. On distille sous pression atmosphérique 50 ml d'éthanol, puis on ajoute à 70° C 40 ml d'eau et on laisse revenir à température ambiante. Dès que les premiers cristaux apparaissent, on agite pendant 15 heures, puis on chauffe à 45-50° C pendant 3 heures. On refroidit le mélange à 0° C et on maintient à cette température pendant 15 heures. On filtre et on lave le précipité formé par un mélange éthanol/eau (1/1 ; v/v) préalablement refroidi à 0° C. Après sèchage sous vide à 80° C, on obtient 15,14 g d'osanétant, Forme I (rendement 99,6 %).

### EXEMPLE 8

### Osanétant - Forme cristalline I

On opére comme décrit à l'EXEMPLE 1, étape a), et la première partie de l'étape b).

Après distillation de l'azéotrope dichlorométhane/eau, on ajoute de l'éthanol puis on élimine l'azéotrope dichlorométhane/éthanol en maintenant constant le volume par addition d'éthanol. La solution d'éthanol ainsi obtenue est concentrée par distillation jusqu'à contenir 100 g d'osanétant dans 240 ml d'éthanol, soit une concentration pondérale de 34,7 % en osanétant. Cette solution dont la température est voisine de 70°C est diluée par 160 ml d'eau soit une concentration pondérale finale de 22,3 % en osanétant. Cette solution, agitée avec un mobile d'agitation à palettes type "impeller", est refroidie avec une pente de refroidissement linéaire de -15°C/heure. A la température de 40°C, on ensemence le milieu avec 5 % en poids de cristaux d'osanétant (Forme I) et on continue à refroidir avec une rampe de refroidissement de -5°C/heure, jusqu'à 20°C. On maintient le milieu à 20°C pendant 4 heures. La suspension d'osanétant ainsi obtenue est réchauffée avec une rampe de chauffage de + 14°C/heure jusqu'à 48°C, puis maintenue à 48°C pendant 2 heures. On refroidit cette suspension jusqu'à 20°C avec une rampe de refroidissement de -5°C/heure. On maintient 20°C pendant 4 heures puis on filtre les cristaux formés. On les lave avec le mélange éthanol/eau (60/40 ; v/v) puis on sèche sous vide à 80°C. On obtient ainsi l'osanétant. Forme cristalline I avec un rendement supérieur à 90 %.

## Revendications

1. Procédé de cristallisation de l'osanetant, **caractérisé en ce que** :
i) l'osanetant contenant moins de 20 % d'impuretés est cristallisé dans un mélange éthanol/eau ou de l'isopropanol pour donner la Forme cristalline I ;
ii) l'osanetant contenant moins de 20 % d'impuretés est cristallisé dans un mélange éthanol/éther isopropylique/eau pour donner la Forme cristalline II.

2. Procédé selon la revendication 1 **caractérisé en ce que** l'osanetant à cristalliser contient moins de 10 % d'impuretés.

3. Procédé de préparation de l'osanetant - Forme cristalline I, selon la revendication 1 ou la revendication 2, **caractérisé en ce que** l'osanetant est cristallisé d'un mélange éthanol/eau ou de l'isopropanol.

4. Procédé de préparation de l'osanetant - Forme cristalline II, selon la revendication 1 ou la revendication 2, **caractérisé en ce que** l'osanetant est cristallisé d'un mélange éthanol/éther isopropylique/eau.

5. Procédé selon la revendication 1 ou la revendication 2 **caractérisé en ce que** :
- soit on ajoute de l'eau à une solution d'osanetant dans l'éthanol et on chauffe à une température inférieure ou égale à la température de reflux du solvant puis on refroidit pour obtenir l'osanetant - Forme cristalline I ;
- soit on chauffe une solution d'osanetant dans l'isopropanol à une température inférieure ou égale à la température de reflux du solvant puis on refroidit pour obtenir l'osanetant - Forme cristalline I ;
- soit on ajoute de l'éther isopropylique et de l'eau à une solution d'osanetant dans l'éthanol, on chauffe à une température inférieure ou égale à la température de reflux du solvant et on refroidit pour obtenir l'osanetant - Forme cristalline II.

6. Procédé selon la revendication 5 pour la préparation de l'osanetant - Forme cristalline I **caractérisé en ce que** :
- soit on ajoute de l'eau à une solution d'osanetant dans l'éthanol et on chauffe à une température de 60°C à 75°C puis on refroidit ;
- soit on chauffe une solution d'osanetant dans l'isopropanol à 60°C-80°C et on refroidit.

7. Procédé selon la revendication 6, **caractérisé en ce que** l'on ajoute de l'eau à une solution d'osanetant dans l'éthanol, on chauffe à une température de 60°C à 75°C, puis on refroidit.

8. Procédé selon la revendication 7, **caractérisé en ce que** l'on refroidit à 20°-25°C puis, soit on amorce la cristallisation, soit on attend que les premiers cristaux apparaissent et, ensuite, on augmente la température jusqu'à 45-50°C, puis on refroidit jusqu'à 0°C et on maintient à cette température.

9. Procédé selon la revendication 7, **caractérisé en ce que** l'on refroidit jusqu'à une température voisine de 40°C, on amorce la cristallisation, on continue à refroidir jusqu'à 20-25°C, on réchauffe la suspension d'osanetant formée jusqu'à 45-50°C, puis on refroidit jusqu'à 20-25°C.

10. Procédé selon la revendication 6, **caractérisé en ce que** l'on chauffe une solution d'osanetant dans l'isopropanol à une température de 60-80°C, puis on refroidit.

11. Procédé selon la revendication 10, **caractérisé en ce que** l'on refroidit jusqu'à une température comprise entre 0 et 50°C puis on amorce la cristallisation, ensuite, on refroidit jusqu'à 0°C et on maintient à 0°C.

12. Procédé selon la revendication 5, pour la préparation de l'osanetant - Forme cristalline II, **caractérisé en ce que** l'on ajoute de l'éther isopropylique et de l'eau à une solution d'osanetant dans l'éthanol, on chauffe à reflux, puis on refroidit dans un mélange éthanol/éther isopropylique, on ajoute de l'éther isopropylique et de l'eau, on chauffe à reflux, puis on refroidit.

13. Procédé selon la revendication 12 **caractérisé en ce que** l'on chauffe à reflux une solution d'osanetant dans un mélange éthanol/éther isopropylique, on ajoute de l'éther isopropylique et de l'eau, on laisse refroidir jusqu'à 40-50°C, puis soit on amorce la cristallisation, soit on attend que les premiers cristaux apparaissent et on refroidit ensuite jusqu'à 20-25°C.

14. Procédé selon l'une quelconque des revendications 1 à 13, **caractérisé en ce que** l'osanetant à cristalliser est préparé par neutralisation de son benzènesulfonate.

15. Osanetant - Forme cristalline I, susceptible d'être obtenu par un procédé selon l'une quelconque des revendications 1, 2, 3, 5 à 11 et **caractérisé en ce qu'**il présente :
- une température de fusion avec un pic dont le maximum est à 143,6 ± 0,5 °C
- une enthalpie de fusion de 68,5 ± 0,5 J/g.

16. Osanetant - Forme cristalline II, susceptible d'être obtenu par un procédé selon l'une quelconque des revendications 1, 2, 4, 5, 12 ou 13 et **caractérisé en ce qu'**il présente :
- une température de fusion avec un pic dont le maximum est à 141,8 ± 0,5 °C
- une enthalpie de fusion de 65,0 ± 0,5 J/g.

17. Osanetant - Forme cristalline I selon la revendication 15, **caractérisé en ce que** son diffractogramme des rayons X de la poudre présente des raies caractéristiques en 2 thêta de Bragg à environ 17,81° ; 11,04° et16,84° .

18. Osanetant - Forme cristalline II selon la revendication 16, **caractérisé en ce que** son diffractogramme des rayons X de la poudre présente des raies caractéristiques en 2 thêta de Bragg à environ 18,35° ; 18,58° et 18,97°.

19. Composition pharmaceutique contenant, en tant que principe actif, un composé selon l'une quelconque des revendications 15 ou 17.

20. Composition pharmaceutique contenant, en tant que principe actif, un composé selon l'une quelconque des revendications 16 ou 18.

21. Composition selon la revendication 19 ou la revendication 20, **caractérisée en ce qu'**elle est sous la forme d'unité de dosage contenant 0,5 à 500 mg de principe actif.

22. Composition selon la revendication 21, **caractérisée en ce que** ladite unité de dosage contient de 1 à 250 mg de principe actif.

## Claims

1. Process for the crystallization of osanetant, **characterized in that**:
i) osanetant comprising less than 20% of impurities is crystallized from an ethanol/water mixture or from isopropanol to give the crystalline form I;
ii) osanetant comprising less than 20% of impurities is crystallized from an ethanol/isopropyl ether/water mixture to give the crystalline form II.

2. Process according to Claim 1, **characterized in that** the osanetant to be crystallized comprises less than 10% of impurities.

3. Process for the preparation of osanetant - crystalline form I according to Claim 1 or Claim 2, **characterized in that** osanetant is crystallized from an ethanol/water mixture or from isopropanol.

4. Process for the preparation of osanetant - crystalline form II according to Claim 1 or Claim 2, **characterized in that** osanetant is crystallized from an ethanol/isopropyl ether/water mixture.

5. Process according to Claim 1 or Claim 2, **characterized in that**:
- either water is added to a solution of osanetant in ethanol and heating is carried out to a temperature of less than or equal to the reflux temperature of the solvent and then cooling is carried out in order to obtain osanetant - crystalline form I;
- or a solution of osanetant in isopropanol is heated to a temperature of less than or equal to the reflux temperature of the solvent and then cooling is carried out in order to obtain osanetant - crystalline form I;
- or isopropyl ether and water are added to a solution of osanetant in ethanol, heating is carried out to a temperature of less than or equal to the reflux temperature of the solvent and cooling is carried out in order to obtain osanetant - crystalline form II.

6. Process according to Claim 5 for the preparation of osanetant - crystalline form I, **characterized in that**:
- either water is added to a solution of osanetant in ethanol and heating is carried out to a temperature of 60°C to 75°C and then cooling is carried out;
- or a solution of osanetant in isopropanol is heated to 60°C-80°C and cooling is carried out.

7. Process according to Claim 6, **characterized in that** water is added to a solution of osanetant in ethanol, heating is carried out to a temperature of 60 to 75°C and then cooling is carried out.

8. Process according to Claim 7, **characterized in that** cooling is carried out to 20-25°C, then either the crystallization is initiated or there is a wait for the first crystals to appear, and, subsequently, the temperature is increased to 45-50°C, cooling is then carried out to 0°C and this temperature is maintained.

9. Process according to Claim 7, **characterized in that** cooling is carried out to a temperature in the region of 40°C, the crystallization is initiated, cooling is continued to 20-25°C, the osanetant suspension formed is reheated to 45-50°C and then cooling is carried out to 20-25°C.

10. Process according to Claim 6, **characterized in that** a solution of osanetant in isopropanol is heated to a temperature of 60-80°C and then cooling is carried out.

11. Process according to Claim 10, **characterized in that** cooling is carried out to a temperature of between 0 and 50°C, the crystallization is then initiated, cooling is subsequently carried out to 0°C and the mixture is maintained at 0°C.

12. Process according to Claim 5 for the preparation of osanetant - crystalline form II, **characterized in that** isopropyl ether and water are added to a solution of osanetant in ethanol, heating is carried out to reflux, then cooling is carried out in an ethanol/isopropyl ether mixture, isopropyl ether and water are added, heating is carried out to reflux and then cooling is carried out.

13. Process according to Claim 12, **characterized in that** a solution of osanetant in an ethanol/isopropyl ether mixture is heated to reflux, isopropyl ether and water are added, cooling to 40-50°C is allowed to take place, then either the crystallization is initiated or there is a wait for the first crystals to appear, and cooling is subsequently carried out to 20-25°C.

14. Process according to any one of Claims 1 to 13, **characterized in that** the osanetant to be crystallized is prepared by neutralization of its benzenesulfonate.

15. Osanetant - crystalline form I, capable of being obtained by a process according to any one of Claims 1, 2, 3 and 5 to 11 and **characterized in that** it exhibits:
- a melting temperature with a peak with a maximum at 143.6 ± 0.5°C
- an enthalpy of fusion of 68.5 ± 0.5 J/g.

16. Osanetant - crystalline form II, capable of being obtained by a process according to any one of Claims 1, 2, 4, 5, 12 or 13 and **characterized in that** it exhibits:
- a melting temperature with a peak with a maximum at 141.8 ± 0.5°C
- an enthalpy of fusion of 65.0 ± 0.5 J/g.

17. Osanetant - crystalline form I according to Claim 15, **characterized in that** its powder X-ray diffractogram exhibits characteristic Bragg 2θ lines at approximately 17.81°, 11.04° and 16.84°.

18. Osanetant - crystalline form II according to Claim 16, **characterized in that** its powder X-ray diffractogram exhibits characteristic Bragg 2θ lines at approximately 18.35°, 18.58° and 18.97°.

19. Pharmaceutical composition comprising, as active principle, a compound according to either one of Claims 15 and 17.

20. Pharmaceutical composition comprising, as active principle, a compound according to either one of Claims 16 and 18.

21. Composition according to Claim 19 or Claim 20, **characterized in that** it is in the form of a dosage unit comprising 0.5 to 500 mg of active principle.

22. Composition according to Claim 21, **characterized in that** said dosage unit comprises from 1 to 250 mg of active principle.

## Patentansprüche

1. Verfahren zur Kristallisation von Osanetant, **dadurch gekennzeichnet, daß** man:
i) Osanetant, das weniger als 20 % Verunreinigungen enthält, in einer Ethanol/Wasser-Mischung oder in Isopropanol kristallisiert zur Bildung der Kristallform I;
ii) man Osanetant, das weniger als 20 % Verunreinigungen enthält, in einer Ethanol/Isopropylether/Wasser-Mischung kristallisiert zur Bildung der Kristallform II.

2. Verfahren nach Anspruch 1, **dadurch gekennzeichnet, daß** das zu kristallisierende Osanetant weniger als 10 % Verunreinigungen enthält.

3. Verfahren zur Herstellung von Osanetant in der Kristallform I nach Anspruch 1 oder Anspruch 2, **dadurch gekennzeichnet, daß** man Osanetant in einer Ethanol/Wasser-Mischung oder in Isopropanol kristallisiert.

4. Verfahren zur Herstellung von Osanetant in der Kristallform II nach Anspruch 1 oder Anspruch 2, **dadurch gekennzeichnet, daß** man Osanetant in einer Ethanol/Isopropylether/Wasser-Mischung kristallisiert.

5. Verfahren nach Anspruch 1 oder Anspruch 2, **dadurch gekennzeichnet, daß** man:
- entweder Wasser zu einer Lösung von Osanetant in Ethanol zugibt und auf eine Temperatur, die gleich oder niedriger liegt als die Rückflußtemperatur des Lösungsmittels, erhitzt und dann abkühlt unter Erhalt von Osanetant in der Kristallform I;
- oder eine Lösung von Osanetant in Isopropanol auf eine Temperatur erhitzt, die gleich oder niedriger liegt als die Rückflußtemperatur des Lösungsmittels, erhitzt und dann abkühlt unter Erhalt von Osanetant in der Kristallform I;
- oder Isopropylether und Wasser zu einer Lösung von Osanetant in Ethanol zugibt und auf eine Temperatur, die gleich oder niedriger liegt als die Rückflußtemperatur des Lösungsmittels, erhitzt und abkühlt unter Erhalt von Osanetant in der Kristallform II.

6. Verfahren nach Anspruch 5 für die Herstellung von Osanetant in der Kristallform I, **dadurch gekennzeichnet, daß** man:
- entweder Wasser zu einer Lösung von Osanetant in Ethanol zugibt und auf eine Temperatur von 60°C bis 75°C erhitzt und dann abkühlt;
- oder eine Lösung von Osanetant in Isopropanol auf 60°C-80°C erhitzt und dann abkühlt.

7. Verfahren nach Anspruch 6, **dadurch gekennzeichnet, daß** man Wasser zu einer Lösung von Osanetant in Ethanol zugibt und auf eine Temperatur von 60°C bis 75°C erhitzt und dann abkühlt.

8. Verfahren nach Anspruch 7, **dadurch gekennzeichnet, daß** man auf 20°-25°C abkühlt und dann die Kristallisation animpft oder abwartet, bis die ersten Kristalle erscheinen und anschließend die Temperatur bis auf 45-50°C erhöht und dann auf 0°C abkühlt und bei dieser Temperatur hält.

9. Verfahren nach Anspruch 7, **dadurch gekennzeichnet, daß** man bis zu einer Temperatur in der Nähe von 40°C abkühlt, die Kristallisation animpft, das Abkühlen bis auf 20-25°C fortsetzt, die gebildete Osanetant-Suspension wieder auf 45-50°C erhitzt und dann wieder auf 20-25°C abkühlt.

10. Verfahren nach Anspruch 6, **dadurch gekennzeichnet, daß** man eine Lösung von Osanetant in Isopropanol auf eine Temperatur von 60-80°C erhitzt und dann abkühlt.

11. Verfahren nach Anspruch 10, **dadurch gekennzeichnet, daß** man bis zu einer Temperatur zwischen 0 und 50°C abkühlt, dann die Kristallisation animpft, anschließend auf 0°C abkühlt und dann bei 0°C hält.

12. Verfahren nach Anspruch 5 für die Herstellung von Osanetant in der Kristallform II, **dadurch gekennzeichnet, daß** man Isopropylether und Wasser zu einer Lösung von Osanetant in Ethanol zugibt, zum Sieden am Rückfluß erhitzt, dann in einer Ethanol/Isopropylether-Mischung abkühlt, Isopropylether und Wasser zugibt, zum Sieden am Rückfluß erhitzt und dann abkühlt.

13. Verfahren nach Anspruch 12, **dadurch gekennzeichnet, daß** man eine Lösung von Osanetant in einer Ethanol/Isopropylether-Mischung zum Sieden am Rückfluß erhitzt, Isopropylether und Wasser zugibt, auf 40-50°C abkühlen läßt, dann entweder die Kristallisation animpft oder abwartet, bis die ersten Kristalle erscheinen und anschließend auf 20-25°C abkühlt.

14. Verfahren nach irgendeinem der Ansprüche 1 bis 13, **dadurch gekennzeichnet, daß** das zu kristallisierende Osanetant durch Neutralisation seines Benzolsulfonats hergestellt wird.

15. Osanetant in der Kristallform I, erhältlich nach einem Verfahren gemäß irgendeinem der Ansprüche 1, 2, 3 oder 5 bis 11, **dadurch gekennzeichnet, daß** es:
- eine Schmelztemperatur mit einem Peak, dessen Maximum bei 143,6 ± 0,5°C liegt, und
- eine Schmelzenthalpie von 68,5 ± 0,5 J/g aufweist.

16. Osanetant in der Kristallform II, erhältlich nach einem Verfahren gemäß irgendeinem der Ansprüche 1, 2, 4, 5, 12 oder 13, **dadurch gekennzeichnet, daß** es:
- eine Schmelztemperatur mit einem Peak, dessen Maximum bei 141,8 ± 0,5 °C liegt, und
- eine Schmelzenthalpie von 65,0 ± 0,5 J/g aufweist.

17. Osanetant in der Kristallform I nach Anspruch 15, **dadurch gekennzeichnet, daß** sein Pulver-Röntgenstrahlenbeugungsdiagramm charakteristische 2 Theta-Banden nach Bragg bei etwa 17,81°, 11,04° und 16,84° aufweist.

18. Osanetant in der Kristallform II nach Anspruch 16, **dadurch gekennzeichnet, daß** sein Pulver-Röntgenstrahlenbeugungsdiagramm charakteristische 2 Theta-Banden nach Bragg bei etwa 18,35°, 18,58° und 18,97° aufweist.

19. Pharmazeutische Zubereitung enthaltend als Wirkstoff eine Verbindung nach einem der Ansprüche 15 oder 17.

20. Pharmazeutische Zubereitung enthaltend als Wirkstoff eine Verbindung nach einem der Ansprüche 16 oder 18.

21. Zubereitung nach Anspruch 19 oder Anspruch 20, **dadurch gekennzeichnet, daß** sie in Einheitsdosisform vorliegt, die 0,5 bis 500 mg des Wirkstoffs enthält.

22. Zubereitung nach Anspruch 21, **dadurch gekennzeichnet, daß** die Einheitsdosis 1 bis 250 mg des Wirkstoffs enthält.
